(19) Europäisches Patentamt
European Patent Office
Office européen des brevets

(11) EP 0 580 763 B1

(12) EUROPEAN PATENT SPECIFICATION

(45) Date of publication and mention of the grant of the patent:
**06.09.2006 Bulletin 2006/36**

(21) Application number: **92911299.3**

(22) Date of filing: **17.04.1992**

(51) Int Cl.:
*C07H 13/04* (2006.01) *A61K 38/47* (2006.01)
*A61K 47/48* (2006.01) *C07K 14/705* (2006.01)
*C07K 16/30* (2006.01)

(86) International application number:
**PCT/US1992/003192**

(87) International publication number:
**WO 1992/018610 (29.10.1992 Gazette 1992/27)**

(54) **COMPOSITIONS AND METHODS FOR ENDOTHELIAL BINDING**

ZUSAMMENSETZUNGEN UND METHODEN FÜR DIE VERKNÜPFUNG VON ENDOTHELZELLEN

COMPOSITIONS ET PROCEDES DE LIAISON ENDOTHELIALE

(84) Designated Contracting States:
**AT BE CH DE DK ES FR GB GR IT LI LU MC NL SE**

(30) Priority: **19.04.1991 US 688037**
**25.06.1991 US 721771**
**26.06.1991 US 721160**

(43) Date of publication of application:
**02.02.1994 Bulletin 1994/05**

(73) Proprietors:
- **THE BOARD OF TRUSTEES OF THE LELAND STANFORD JUNIOR UNIVERSITY**
**Stanford, California 94305 (US)**
- **Magnani, John L.**
**Rockville, MD 20853 (US)**

(72) Inventors:
- **MAGNANI, John, L.**
**Rockville, MD 20853 (US)**
- **BUTCHER, Eugene, C.**
**Portola Valley, CA 94025 (US)**
- **BERG, Ellen, L.**
**Fremont, CA 94536 (US)**

(74) Representative: **Brown, John D. et al**
**FORRESTER & BOEHMERT**
**Pettenkoferstrasse 20-22**
**80336 München (DE)**

(56) References cited:
**EP-A- 0 381 310** **US-A- 4 851 511**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

### Technical Field

The present invention is generally directed toward the modulation of leukocyte homing to provide therapies for inflammation and other pathogenic conditions associated with leukocyte infiltration into tissue; and toward the inhibition of cancer metastasis mediated by endothelial adhesion molecules, and more specifically, toward such inhibition through the use of saccharides, glycoconjugates, antibodies, and other agents, which disrupt such binding of cancer cells to endothelia.

### Background of the Invention

The bloodstream is the pathway for numerous cells which migrate throughout the body, monitoring conditions. Cells of the lymphoid and myelomonocytic lineages act to identify foreign substances, such as pathogens, aberrant cells, and some compounds, and remove them from the system. These cells have available a large variety of mechanisms for protecting the host from the foreign substance. Many of these mechanisms are highly destructive and result in cytotoxicity of native tissue, inflammation, degradation, and the like. Mechanisms may involve the production of superoxide, secretion of various degradative compounds, such as perforins, endocytosis, etc.

While in many situations these protective mechanisms are salutary, in many other situations, they are found to have detrimental effects, involving inflammatory lesions, such as myocarditis, inflammatory bowel disease, psoriasis, allergic contact dermatitis, lichen planus, lymphoid hyperplasia in skin, inflamed synovia, reperfusion injury, etc.

In recent years, it has been shown that the migrating cells have specific surface membrane proteins associated with their homing or being directed to a particular site. Specialized venules including the high endothelial venules, serve as beacons for these cells, expressing proteins referred to as endothelial leukocyte adhesion molecules and addressins, which bind to the "homing receptors" or adhesion molecule surface membrane proteins of the migrating cells. After binding to the venules, the cells migrate by diapedesis, by mechanisms unknown, to the site of inflammation or injury.

Due to the difficulties in the current approaches in the treatment and prevention of diseases associated with or aggravated by the infiltration of migrating cells into an inflamed site, there is a need in the art for improved compositions and methods for inhibiting cell infiltration. The present invention fills this need, and further provides other related advantages.

One such related advantage pertains to cancer. Despite enormous investments of financial and human resources, cancer remains one of the major causes of death. Current cancer therapies cure only about fifty percent of the patients who develop a malignant tumor. In most human malignancies, metastasis is the major cause of death.

Metastasis is the formation of a secondary tumor colony at a distant site. It is a multistep process of which tumor invasion is an early event. Tumor cells locally invade host tissue barriers, such as the epithelial basement membrane, to reach the interstitial stroma, where they gain access to blood vessels ("hematogeneous metastasis") or lymphatic channels for further dissemination. After invading the endothelial layer of a vessel wall, the circulating tumor cells are dislodged into the circulation and arrest in the precapillary venules of the target organ by adherence to endothelial cell lumenal surfaces, or exposed basement membranes. The tumor cells again invade the vascular wall to enter the organ parenchyma. Finally, the extravasated tumor cell grows in a tissue different from where it originated.

Most cancer cells fail to survive in the circulation and it appears that normally the lining of blood vessels acts as a barrier to tumor cell extravasation. Endothelial injury or perturbation increases tumor metastasis. In addition, certain factors, such as cytokines, have been shown to substantially increase the adhesion of cancer cells to treated endothelium in vitro. Interleukin 1 (IL-1) and tumor necrosis factor (TNF), which are cytokines, each stimulate the biosynthesis and expression of a cell surface receptor called ELAM-1 (endothelial leukocyte adhesion molecule). ELAM-1 is a member of a family of calcium-dependent cell adhesion receptors, known as selectins or selectins, which includes LECAM-1 and GMP-140 (also known as PADGEM or CD62). During an inflammatory response, ELAM-1 on endothelial cells functions as a "homing receptor" for leukocytes. Recently, ELAM-1 on endothelial cells was shown to mediate the increased adhesion of colon cancer cells to endothelium treated with cytokines (Rice and Bevilacqua, Science 246:1303-1306, 1989).

In most human malignancies, distant metastases are often too small to be detected at the time the primary tumor is treated. Furthermore, widespread initiation of metastatic colonies usually occurs before clinical symptoms of metastatic disease are evident. The size and age variation in metastases, their dispersed anatomical location, and their heterogeneous composition are all factors that hinder surgical removal and limit the concentration of anticancer drugs that can be delivered to the metastatic colonies. It has been estimated, for example, that in 1991 there will be over 60,000 deaths and over 150,000 new cases from just colorectal cancer in the U.S. alone.

Cell, 1990, vol. 63, p.861-863 discloses carbohydrate ligands of the LEC cell adhesion molecules.

Science, 1990, vol. 250, p. 1130-1132 discloses that cell adhesion by ELAM -1 is mediated by the carbohydrate

ligand, sialyl-$Le^x$.

Science, 1990, vol. 250, p. 1132-1135 discloses that binding of myeloid cells to soluble ELAM-1 is inhibited by a monoclonal antibody recognizing sialyl-$Le^x$ or by proteins bearing sialyl-$Le^x$.

WO-A-9013300 discloses DNA sequences encoding endothelial cell-leukocyte adhesion molecules ELAMs, and methods for producing such molecules.

Cell, 1990, vol. 63, p. 475-484 discloses that transfection of a human fucosyltransferase cDNA into nonmyeloid cell lines confers ELAM 1 - dependent endothelial adhesion.

Int. J. Cancer, 1990, vol. 45, p. 1022-1027 discloses the purification and characterisation of a peanut - agglutinis - binding pancreatic-cancer-related serum mucus glycepotein.

Due to the difficulties in the current approaches to the treatment and prevention of metastases, there is a need in the art for improved compositions and methods for inhibiting metastasis mediated by endothelial adhesion molecules. The present invention fills this need, and further provides other related advantages.

## Summary of the Invention

In accordance with the present invention there is provided a composition set forth in claim 1 to 6.

There is also provided the composition set forth in any one of claim 1 to 6 for use in therapy.

Briefly stated, the present invention provides compositions for modulating the leukocyte binding involving selectins to endothelial cells as sites of leukocyte exit from the blood. The compositions are characterized by binding to the selectin ELAM-1 or other selectin, and are at least in part other than polypeptide and substantially free of the natural polypeptide associated with the homing receptor, e.g., the cutaneous lymphocyte-associated antigen or LECAM-1. These compositions find particular use in inhibiting the homing of leukocytes, particularly lymphocytes, to sites of inflammation.

In one aspect, there is disclosed a method for modulating the binding of leukocytes or platelets to endothelial cells, the method comprising: adding to a combination of cells comprising leukocytes and endothelial cells expressing selectins or carbohydrate ligands thereof, in an amount sufficient to modulate the binding of leukocytes to endothelial cells, a compound capable of being cross-reactive and/or competitive with sialyl-$Le^x$, sialyl-$Le^a$ or the cutaneous lymphocyte-associated antigen in binding to a selectin, wherein the compound is other than sialyl-$Le^x$ when the selectin is ELAM-1. In one embodiment, the compound comprises sialic acid and fucopyranose bonded to a group comprising a conformationally constrained chain of at least 2 atoms.

In another aspect, there is disclosed a compound other than sialyl-$Le^x$ comprising Neu5Acα2-3Galβ1-x[Fucα1-y]GlcNAc, wherein one of x and y is 3 and the other 4, or derivative thereof capable of binding to ELAM-1, LECAM-1 or GMP-140 for use within a method for inhibiting the infiltration of leukocytes into an inflammation site of a host. In one embodiment, there is disclosed a compound comprising Neu5Acα2-3Galβ1-x[Fucα1-y]GlcNAc, wherein one of x and y is 3 and the other 4, or derivative thereof capable of binding to a selectin for use within a method for inhibiting the infiltration of lymphocytes into an inflammation site of a host.

In yet another aspect, there is disclosed a compound comprising Neu5Acα2-3Galβ1-x[Fucα1-y]GlcNAc, wherein one of x and y is 3 and the other 4, or derivative thereof capable of binding to a selectin for use within a method for inhibiting the binding of platelets to endothelial cells.

In a related aspect, novel compounds are disclosed. In one embodiment, the compound comprises a compound other than a naturally occurring sialyl-$Le^a$ or sialyl-$Le^x$ antigen comprising: sialic acid and fucopyranose bonded to a group comprising a conformationally constrained chain. In another embodiment, the compound comprises a compound other than a naturally occurring sialyl-$Le^a$ or sialyl-$Le^x$ antigen comprising: Neu5Acα2-3Galβ1-x[Fucα1-y]GlcNAc, wherein one of x and y is 3 and the other 4, or derivative thereof capable of binding to a selectin. In yet another embodiment, the compound comprises a compound other than a sialyl-$Le^x$ or sialyl-$Le^a$ antigen comprising: Neu5Aca2-3Galβ1-x [Fucα1-y]R, wherein one of x and y is 3 and the other 4, R is a linker such as a saccharide or derivative, including ringed compounds such as constrained ring compounds, the compound being capable of binding to a

In addition, there are disclosed compositions and methods for the inhibition of cancer metastasis mediated by endothelial adhesion molecules. In one aspect, there are disclosed methods for inhibiting, within a biological preparation, the binding of malignant cells expressing sialyl-$Le^a$, di-sialyl-$Le^a$ or sialyl-$Le^x$ to endothelial cells expressing a selectin such as ELAM-1; or the binding of malignant cells expressing sialyl-$Le^a$ or di-sialyl-$Le^a$, to endothelial cells expressing a selectin such as ELAM-1. In one embodiment, the method comprises incubating the biological preparation with at least one agent that inhibits the binding of malignant cells expressing sialyl-$Le^a$, di-sialyl-$Le^a$ or sialyl-$Le^x$, to endothelial cells expressing a selectin, wherein said agent is other than sialyl-$Le^x$ when said malignant cells express sialyl-$Le^x$. In another embodiment, the method comprises incubating the biological preparation with at least one agent that inhibits the binding of malignant cells expressing sialyl-$Le^a$, di-sialyl-$Le^a$ or sialyl-$Le^x$, to endothelial cells expressing ELAM-1, wherein said agent is other than sialyl-$Le^x$ when said malignant cells express sialyl-$Le^x$. In another embodiment, the method comprises incubating malignant cells expressing sialyl-$Le^a$ or di-sialyl-$Le^a$ with at least one enzyme inhibitor

that inhibits the biosynthesis of sialyl-$Le^a$ or di-sialyl-$Le^a$ by the malignant cells. In yet another embodiment, the method comprises incubating said malignant cells with at least one enzyme that alters sialyl-$Le^a$ or di-sialyl-$Le^x$ of said malignant cells such that said malignant cells are incapable of binding to a selectin.

There are disclosed in another aspect, compositions for use in methods for inhibiting the spread of malignant cells expressing sialyl-$Le^a$, di-sialyl-$Le^a$ or sialyl-$Le^x$, to secondary sites in a warm-blooded animal. In one embodiment, the composition ses an agent that inhibits the binding of malignant cells expressing sialyl-$Le^a$, di-sialyl-$Le^a$ or sialyl-$Le^x$, to endothelial cells expressing a selectin. In another embodiment involving hematogeneous metastasis, the composition comprises an agent that inhibits the binding of malignant cells expressing sialyl-$Le^a$, di-sialyl-$Le^a$ or sialyl-$Le^x$, to endothelial cells expressing ELAM-1. In a related aspect, compositions are provided for use in methods for inhibiting the spread of malignant cells expressing sialyl-$Le^a$ or di-sialyl-$Le^a$, to secondary sites in a warm-blooded animal. In one embodiment, the composition comprises an enzyme inhibitor that inhibits the biosynthesis of sialyl-$Le^a$ or di-sialyl-$Le^a$ by the malignant cells. In another embodiment, the composition comprises an enzyme that alters sialyl-$Le^a$ or di-sialyl-$Le^a$ of malignant cells expressing sialyl-$Le^a$ or di-sialyl-$Le^a$ such that malignant cells are incapable of binding to a selectin.

In another aspect, methods are disclosed for inhibiting within a biological preparation the binding of malignant cells expressing sialyl-$Le^a$, di-sialy1-$Le^a$ or sialyl-$Le^x$, to endothelial cells. In one disclosed embodiment, the method comprises incubating a biological preparation, containing endothelial cells expressing a selectin, with at least one agent capable of reacting with both sialyl-$Le^a$ and sialyl-$Le^x$. In another disclosed embodiment, the method comprises incubating a biological preparation, containing endothelial cells expressing ELAM-1, with at least one agent capable of reacting with both sialyl-$Le^a$ and sialyl-$Le^x$.

In another related aspect, compositions are provided for use in methods for inhibiting the spread of malignant cells expressing sialyl-$Le^a$, di-sialyl-$Le^a$ or sialyl-$Le^x$, to secondary sites in a warm-blooded animal. In one embodiment, the composition comprises an agent capable of reacting with both sialyl-$Le^a$ and sialyl-$Le^x$. In another embodiment involving hematogeneous metastasis, the composition comprises an agent capable of reacting with both sialyl-$Le^a$ and sialyl-$Le^x$.

These and other aspects of the present invention will become evident upon reference to the following detailed description and attached drawings.

**Brief Description of the Drawings**

Figure 1 is a graphic depiction of models for sialyl-$Le^a$ and sialyl-$Le^x$.
Figure 2 describes pictorially a cell binding assay used to assess binding of human ELAM-1 transfected mouse cells to neoglycoproteins.
Figure 3 graphically illustrates the relative binding of human ELAM-1 transfected mouse cells to certain neoglycoproteins.
Figure 4 graphically illustrates the relative binding of human ELAM-1 transfected mouse cells to certain neoglycoproteins.
Figure 5 graphically illustrates the inhibition of binding of human ELAM-1 transfected mouse cells to immobilized sialyl-$Le^a$-HSA (human serum albumin) by soluble sialyl-$Le^a$-HSA.
Figure 6 graphically illustrates the inhibition of binding of human ELAM-1 transfected mouse cells to immobilized sialyl-$Le^a$-PA (polyacrylamide) by soluble sialyl-$Le^a$-PA.
Figure 7 graphically illustrates the selective binding of LECAM-1 transfected cells to neoglycoproteins.
Figure 8 graphically illustrates the binding of lymphocytes to neoglycoproteins.

Detailed Description of the Invention

Prior to setting forth the invention, it may be helpful to an understanding thereof to set forth definitions of certain terms to be used hereinafter.

ELAM-1 (also known as "E-selectin") is a vascular selectin. ELAM-1 recruits neutrophils during acute inflammation, but during chronic inflammation is selectively found in skin, binding skin homing lymphocytes, i.e., it doubles as a skin vascular addressin.

GMP-140 (also known as "G-selectin") is a vascular selectin which binds neutrophils and monocytes early in inflammation; it is also expressed on stimulated platelets.

LECAM-1 (also known as "L-selectin") is a vascular selectin involved in neutrophil, monocyte, etc., extravasation in acute inflammation, and lymphocyte homing to peripheral lymph nodes and some sites of chronic inflammation.

Selectins (formerly referred to as "LEC-CAMs") are defined structurally, being a lectin with the same or similar structural motifs as LECAM-1 (the Mel-14 antigen).

Addressins are defined as any tissue specific vascular adhesion molecule involved in lymphocyte homing. The peripheral lymph node addressin (PLN) is a glycoprotein (carbohydrate ligand) for the lymph node homing receptor (selectin) LECAM-1. The mucosal addressin is a 60kD glycoprotein.

Antibody, as used herein, includes both monoclonal and polyclonal antibodies and may be an intact molecule, a fragment thereof, or a functional equivalent thereof. The antibody may be genetically engineered. Examples of antibody fragments include $F(ab')_2$, Fab', Fab and Fv.

Saccharide, as used herein, includes oligosaccharides, and may be naturally derived, synthetically prepared, portions of either, and derivatives of any of the foregoing.

Glycoconjugate, as used herein, includes a saccharide which is linked to a non-saccharide molecule, e.g., a lipid or a polypeptide.

As noted above, there is dislosed herein the prophylactic and therapeutic modulation of homing of leukocytes, particularly lymphocytes, to sites of inflammation. The compositions are characterized by binding to an endothelial cell or leukocyte lectin adhesion molecules or if the selectin family are cross-reactive with at least one epitope of sialyl-$Le^x$, and sialyl-$Le^a$, are other than sialyl-$Le^x$ and will usually involve at least about three saccharide monomer units.

The active structures of the compositions which find use will be under about 5,000 molecular weight and may be under about 3,000 molecular weight, generally being at least about 800 molecular weight. The compositions themselves may have multiple copies of the active structures, bonded to a common backbone (polymeric chain such as polyacrylamide, polyvinylalcohol, cyclodextrans, etc.), liposomes, and the like. Any compound which has the above-indicated characteristics of cross-reactivity in binding to at least one of ELAM-1, LECAM-1, or GMP-140, is other than sialyl-$Le^x$ or protein conjugate thereof and is physiologically and pharmacokinetically acceptable may be employed. The compounds may be naturally occurring or synthetic and may be saccharides, synthetic organic compounds or the like.

Of particular interest are the sugars sialic acid (neuraminic acid), galactose, fucose, or derivatives thereof, combined to form an oligosaccharide derivative. The sugar monomers may be further derivatized by having up to four, usually not more than three groups bound to carbon, nitrogen or oxygen, which groups may include an additional sugar, such as sialic acid, glucosamine, galactose, glucose, fucose, etc., alkyl groups, such as methyl, ethyl, acyl groups, such as acetyl, etc., and the like. The site of substitution will not interfere with the binding of the compound to its complementary receptor or lectin domain but may provide such advantages as improved pharmacokinetics, stability, ease of synthesis, reduced toxicity, enhanced affinity, and the like.

Leukocytes which may be modulated as to their homing to tissues, where the leukocyte or endothelial cell is expressing the selectin, include neutrophils, T-lymphocytes and B-lymphocytes, platelets, etc. These cells are found to home to a variety of injured, diseased, or otherwise pathogenic states, particularly associated with inflammation. Infiltration of these cells can be associated with such conditions as psoriasis, allergic contact dermatitis, lichen planus, lymphoid hyperplasia in the skin, non-specific chronic dermatitis, pityriasis lichenoids et varioformis acuta, granuloma annular, cutaneous drug eruption, pityriasis rubra pilaris, inflamed synovia, reperfusion injury, or the like. Sites to which the leukocytes migrate include peripheral lymph nodes, skin, Peyers patches, spleen, mesenteric lymph nodes (mucosal tissue), synovium, and other lymphoid and extralymphoid tissues and sites of inflammation.

The subject compositions may be prepared in accordance with conventional ways or isolated from a natural source, e.g., milk. Descriptions of the preparations of sialyl-$Le^x$, sialyl-$Le^a$, the common portions of the two compounds, namely Neu5Ac2, 3Galβ-x[Fucα1-y]G1cNAc, wherein one of x and y is 3, and the other is 4, and cross-reactive derivatives thereof are illustrated by the synthesis of a variety of sugars which may be found in Paulsen, (1982) Angew. chem. Int. Ed. 94:184; Fugedi et al., (1987) Glycoconjugate J. 4:97; and Okamoto and Goto, (1990) Tetrahedron 46:5835; Kameyama et al., (1991) Carbohydr. Res. 209:Cl; and Palcic et al., (1989) Carbohydr. Res. 190:1-11.

The novel compounds of this invention will be other than the naturally occurring sialyl-$Le^a$ or sialyl-$Le^x$ antigens found as polysaccharide markers on human cells. The compounds are characterized by having a structure which comprises, or is immunologically cross-reactive with a structure that comprises, a fucopyranose and a sialic or derivative thereof in a spatial conformation associated with both sialyl-$Le^a$ and sialyl-$Le^x$. Thus, the two sugars, sialic acid and fucopyranose, will be bonded to a chain which permits the sugars to assume the proper orientation and spatial conformation, preferably provides restraint in maintaining such conformation.

The backbone chain may be from 10 to 20, usually 3 to 8, preferably 3 to 7, more preferably 5 to 6 atoms, which may be carbon, nitrogen or oxygen, and may involve alicyclic, cyclic, heterocyclic or aromatic units or combinations thereof. Where a sugar is at least a portion of the backbone, desirably the sialic acid group will be present as the non-reducing terminal sugar of a disaccharide, where the other sugar is preferably galactose, and the disaccharide is separated by from about 1 to 4, preferably 1 to 3, particularly 2 atoms, usually carbon and optionally oxygen atoms, from the fucopyranose. The group serving as the separating chain desirably will be conformationally constrained, particularly as a cyclic or heterocyclic group. The group may be substituted with one or more oxy (including hydroxy) groups. By conformationally constrained for cyclic groups are intended ranges of from 3 to 7, usually 5 to 6 annular members, or sterically hindered compounds, or other structured where the atoms of the chain are inhibited from free rotation. The sialyl and fucopyranose groups may be cis or trans, equatorial or polar, in their spatial positions, usually trans.

For the most part, the subject compositions have as their core structure:

Neu5Acα2-3Galβ1-x[Fucα1-y]R

wherein R is glucose or derivatives, e.g., glucosamine, N-acetyl glucosamine, etc., and other ring structures including constrained cyclic structures, where any of the positions of the core structure may be substituted without interfering with the binding to selectins. Sites for substitution include the available positions of galactose, glucose, and fucose, particularly with a sugar, e.g., sialic acid, glucosamine, N-acetyl glucosamine, glucose, neuraminic acid, fucose, disaccharides thereof, etc., where the nitrogen atoms may be alkylated or acylated; and the like.

Of particular interest are compounds comprising a cyclic group to which fucose and a disaccharide with neuraminic acid as the non-reducing terminal sugar is bonded, where the fucose and disaccharide are separated by from 2 to 3, particularly carbon atoms and optimally an oxygen atom. Thus the cyclic compound may be of 5 to 7 annular members, particularly 6 annular members, and may include 1,2-cyclohexanediol, 1,3-cyclohexanediamine, 1,2-cyclohexanolamine, 1,2-cyclopentandiol, 2,3- or 3,4-dihydroxypyran, and the like. The positions may be cis or trans, preferably trans.

For a variety of purposes, the saccharidic compounds may be conjugated to other compounds, such as lipids, detergents, e.g., non-ionic detergents, such as polyaklyleneoxy groups, with alkylene of from 2-3 carbon atoms, usually under about 5kDal, naturally occurring or synthetic organic compounds where the active structure is under about 2kDal, which may be alicyclic, aromatic, acyclic or heterocyclic, polymeric compounds, such as physiologically acceptable polymers, e.g., acrylates, proteins, or the like which may be under about 100kD or more.

Proteins which may find use as carriers include serum albumin, casein, gelatin, etc. Conjugates may be prepared as immunogens to produce antisera or monoclonal antibodies specific for the binding epitope. Thus, antibodies could be used to inhibit homing of leukocytes, e.g., neutrophils, lymphocytes or other leukocytes. Anti-idiotypic antibodies may be prepared which would compete with the binding epitope for the selectins to prevent lymphocyte infiltration.

The subject compounds may be conjugated to the carriers directly, but more usually through a spacer. Various spacers are known for linking to proteins, particularly spacers incorporating aromatic groups, e.g., phenylene, substituted with from 1 to 2 amino groups where the other functionality may be a carboxylic acid, aldehyde, mercaptan, activated olefin or the like. In bonding the spacer to the saccharide through an amino group, the linkage may provide for retention of the anomeric configuration of the reducing sugars or reductive amination may be employed resulting in an aminoalditol (Kallin et al., Glycoconjugate *J.* 3:311, 1986).

Based on the configuration of the binding epitope, using computer-assisted design, synthetic organic compounds can be devised which would compete with the binding epitope for the addressin.

The subject compositions may be administered in any convenient way, depending upon the particular nature of the composition. Various physiologically acceptable media may be employed, such as deionized water, saline, phosphate buffered saline, aqueous ethanol, and the like. Depending upon the nature of the compound, it may be administered typically, parenterally or orally, subcutaneously, intravascularly, topically, peritoneally, and the like. The particular dosage will vary with the frequency of administration, the manner of administration, the activity of the compound, the indication being treated, and the like.

As noted above, also disclosed herein are compositions and methods for the inhibition of cancer metastasis mediated by endothelial adhesion molecules. More specifically, the disclosure of the present invention shows that antibodies, saccharides, glycoconjugates therefrom, enzymes or enzyme inhibitors may be used to inhibit the binding of malignant cells to endothelial cells for a variety of purposes in vivo and in vitro.

As described above, metastasis is a multistep process. During metastasis, cancer cells circulate through the microvascular and lymph systems and then migrate through the walls of the blood or lymph vessels to establish a new and aggressive tumor at a secondary organ site. A critical step in the metastasis process is the adherence of circulating cancer cells to the endothelial lining of blood vessel or lymph vessel walls. As disclosed within the present invention, the carbohydrates sialyl-$Le^a$ and di-sialyl-$Le^a$, which are expressed at the surface of certain cancer cells, function as a ligand (i.e., binding partner) for selectins, such as ELAM-1, which are expressed at the surface of endothelial cells. Therefore, for those cancer cells, metastasis involves the adherence of cancer cells to the endothelial cells via the binding of sialyl-$Le^a$ and/or di-sialyl-$Le^a$ on the cancer cells to adhesion molecules on endothelial cells. Other cancer cells express predominantly sialyl-$Le^x$, or sialyl-$Le^x$ and sialyl-$Le^a$ (and/or di-sialyl-$Le^a$). The present invention discloses that selectins, such as ELAM-1, bind a carbohydrate domain common to both sialyl-$Le^a$ and sialyl-$Le^x$ on malignant cells, and therefore agents can be produced which are capable of binding to both. Other sialylated glycoconjugates may be expressed as well which possess the common domain.

Inhibition of the initial binding event between selectins and sialylated structures by the methods of the present disclosure prevents the adhesion of metastatic cells to the endothelial lining of blood or lymph vessel walls, thereby eliminating the spread of metastatic cells to secondary organs. Suitable blocking agents include those which inhibit the binding of malignant cells expressing sialyl-$Le^a$, di-sialyl-$Le^a$, or sialyl-$Le^x$ (including or not di-sialyl-$Le^x$), to endothelial

cells expressing selectin adhesion molecules such as ELAM-1. Representative agents include antibodies, saccharides and glycoconjugates therefrom, enzymes and enzyme inhibitors.

The antibodies employed in the present invention may be polyclonal or monoclonal antibodies. Briefly, polyclonal antibodies may be produced by immunization of an animal and subsequent collection of its sera. Immunization is accomplished, for example, by a systemic administration, such as by subcutaneous, intrasplenic or intramuscular injection, into a rabbit, rat or mouse. It is generally preferred to follow the initial immunization with one or more booster immunizations prior to sera collection. Such methodology is well known and described in a number of references.

Monoclonal antibodies (MAbs) suitable within the present disclosure include those of murine or human origin, or chimeric antibodies such as those which combine portions of both human and murine antibodies (i.e., antigen binding region of murine antibody plus constant regions of human antibody). Human and chimeric antibodies may be produced using methods known by those skilled in the art. Human antibodies and chimeric human-mouse antibodies are advantageous because they are less likely than murine antibodies to cause the production of anti-antibodies when administered clinically.

MAbs may be generally produced by the method of Kohler and Milstein (Nature 256: 495-497 , 1975; Eur. J. Immunol. 6:511-519, 1976). Briefly, the lymph nodes and/or spleens of an animal immunized with molecules containing sialyl-$Le^a$ or di-sialyl-$Le^a$ are fused with myeloma cells to form hybrid cell lines ("hybridomas" or "clones"). Each hybridoma secretes a single type of immunoglobulin and, like the myeloma cells, has the potential for indefinite cell division. Hybridomas are selected for producing antibodies that bind the desired carbohydrate structure by screening appropriate neoglycoconjugates. An alternative to the production of MAbs via hybridomas is the creation of MAb expression libraries using bacteriophage and bacteria (e.g., Sastry et al., Proc. Natl. Acad. Sci USA 86:5728, 1989; Huse et al., Science 246:1275, 1989). Selection of antibodies exhibiting appropriate specificity may be performed in a variety of ways which will be evident to those skilled in the art. Typically, such antibodies will selectively bind with an affinity of about $10^7$ liters/mol or higher.

Representative examples of MAbs suitable within the present disclosure include N-19-9 and HECA-452 for sialyl-$Le^a$, and FH-7 for di-sialyl-$Le^a$. MAb N-19-9 is available from ATCC (American Type Tissue Collection, Rockville, Maryland) as ATCC HB 8059 or may be produced as described in U.S. Patent No. 4,471,057 (and Somatic Cell Genet. 5:957-971, 1979; J. Biol. Chem. 257:14365, 1982). MAb HECA-452. may be produced according to Duijvestijn et al., Am. J. Path. 130:147-155, 1988. FH-7 may be produced according to Nudelman et al., J. Biol. Chem. 261:5487, 1986.

In addition to antibodies which are capable of binding to sialyl-$Le^a$, di-sialyl-$Le^a$ or sialyl-$Le^x$, saccharides and glycoconjugates therefrom may also inhibit the binding of metastatic cells expressing sialyl-$Le^a$, di-sialyl-$Le^a$ or sialyl-$Le^x$, to endothelia. As used herein, the terms "sialyl-$Le^a$" and "di-sialyl-$Le^a$" represent structures I and II, respectively, as follows:

```
Neu5Acα2-3Galβ1-3GlcNAcβ1-3Galβ1-R          (I)
                     4
                     |
                   Fucα1


                Neu5Acα2
                     |
                     6
Neu5Acα2-3Galβ1-3GlcNAcβ1-3Galβ1-R          (II)
                     4
                     |
                   Fucα1
```

Neu5Ac represents sialic acid; Gal represents galactose; GlcNAc represents N-acetyl-glucosamine; Fuc represents fucose and R is typically a ceramide (with a glucose residue interposed) or a protein. Sialyl-$Le^x$ is an isomer of sialyl-$Le^a$ wherein the Gal-GlcNAc linkage is β1-4 and the Fuc-GlcNAc linkage is αl→3. Saccharides suitable within the present disclosure include the carbohydrate portion of sialyl-$Le^a$ or di-sialyl-$Le^a$ (i.e., formula I or II minus R), and derivatives of either, including those which cross-react with both sialyl-$Le^a$ and sialyl-$Le^x$. Derivatives of these compounds include substitution of individual saccharide residues with other saccharide residues and/or with non-saccharide molecules such as hexyl rings without hydroxyl groups. For example, the internal GlcNAc may be replaced with another saccharide residue such as a glucose (Glc). Alternatively (or in addition to substitutions), the carbohydrate portion of sialyl-$Le^a$, di-sialyl-Le$^a$, or derivatives thereof, may be truncated by deletion of one or more saccharide residues. For example, a tetrasaccharide may be created with the structure:

```
Neu5Acα2-3Galβ1-3Glc
                  4
                  |
              Fucα1
```

Given the teachings described herein, it will be evident to those skilled in the art that other saccharides will be suitable within the present disclosure.

A saccharide may be coupled to a non-saccharide molecule to form a glycoconjugate. For example, a saccharide may be linked to a polyacrylamide. Alternatively, a saccharide may be linked to a lipid. Typical lipids include ceramide, i.e., sphingolipid bases which are acylated on the amine with a fatty acid. For example, sialyl-Le$^a$, di-sialyl-Le$^a$, or a saccharide cross-reaction with sialyl-Le$^a$ and sialyl-Le$^x$ may be linked to a ceramide. Alternatively, a saccharide may be bonded to an amino acid or an amino acid-containing molecule, such as a peptide, a polypeptide or a protein. Saccharides are naturally linked to an amino acid or amino acid-containing molecule via the hydroxyl group of a serine or threonine amino acid residue, but can also be linked through other groups such as an amino group.

Saccharides and glycoconjugates provided by the present disclosed may be represented by structures III and IV as follows:

```
Neu5Acα2-3Galβ1-3xβ1-3yβ1-4zβ1-R            (III)
                 4
                 |
             Fucα1
```

```
                 Neu5Acα2
                        |
                        6
Neu5Acα2-3Galβ1-3xβ1-3yβ1-4zβ1-R            (IV)
                        4
                        |
                    Fucα1
```

R includes H, OH, lipid, ceramide, or one or more amino acids; x, y and z are independently selected from saccharides, or either y or z or both may be absent.

Numerous methods for preparing saccharides and glycoconjugates are well known to those skilled in the art. Saccharides may be prepared synthetically using chemical, and/or enzymatic, reagents and techniques. For example, sialyl-Le$^a$ saccharides have been prepared by enzymatic synthesis (e.g., Palcic et al., Carbohydr. Res. 190:1-11, 1989). Glycoconjugates may be prepared, for example, through reductive amination. The method of Zopf et al. (Meth. Enzymol. 50:171-175, 1978; Jeffrey et al., Biochem. Biophys. Res. Commun. 62:608-613, 1975) involves 4-aminophenethylamine derivatives of saccharides via reductive amination using sodium borohydride. In brief, sugars are first reacted with the amino reagent by dissolving them in the neat reagent for 15 hours. Sodium borohydride in ethanol is then added. After 5 hours, the product is separated from the reagent by gel filtration and ion exchange chromatography. The derivatives may then be coupled to a molecule containing a group which is reactive with amines. The same amine derivative may be coupled to saccharides using sodium cyanoborohydride. (Svensson et al., J. Immunol. Meth. 25:323-335, 1979). In brief, a sugar is dissolved in water, and the same volume of amine (a 170-fold molar excess) is added together with sodium cyanoborohydride (a ten-fold molar excess). The reduction is performed at pH 8 for 48 hours, and the product purified by gel chromatography. Coupling to different molecules, such as proteins, may be performed by the isothiocyanate coupling method.

Another example of a reagent suitable for preparing glycoconjugates by reductive amination is p-trifluoroacetamidoaniline (TFAN). The reductive amination reaction is carried out in aqueous solution overnight at pH 5-6 with sodium cyanoborohydride as the reducing agent. Typically, a 5-fold excess of TFAN is used. TFAN-derivatized saccharides are generally protected from oxidation by N-acetylation, e.g., by treatment with methanolic acetic anhydride, to yield TFAc-derivatives. Prior to conjugation, the N-trifluoroacetamido protective group is removed by treatment of the TFAc derivative with aqueous ammonia or 0.5 M sodium hydroxide for 3 hours. Conjugation of the derivatives to molecules, for example to proteins such as bovine serum albumin (BSA), may be achieved by isothiocyanate coupling methods. Other examples of suitable reagents and reactions include p-tetradecylaniline derivatives of saccharides and the preparation of aminoalditols by oxidation of saccharide TFAN derivates with cerium ammonium sulfate (Lindenberg et al., J. Reprod. Fert. 89:431-439, 1990).

Multivalent carbohydrate drug candidates can be prepared from N-acrylol glycosylamines which are produced by acylating glycosylamines with acryl chlorides. The N-acrylol glycosylamines are co-polymerized with acrylamide using a radical initiator in aqueous solution to produce multivalent carbohydrate polymers in which the degree of substitution is determined by the molar ratio of the reactants (Kallin et al., J. Carbohydrate Chem. 8:597-611, 1989). Using this method, sialyl-$Le^a$ was co-polymerized to form a multivalent carbohydrate polyacrylamide ("$SLe^a$-PA," e.g., Figure 6). This multivalent carbohydrate drug candidate is non-toxic and water soluble. The molecular weight and hapten density can be determined by altering the ratio of reactants and the reaction time.

The inhibition of the binding of cancer cells expressing sialyl-$Le^a$, di-sialyl-$Le^a$ or sialyl-$Le^x$, to endothelia has a variety of in vitro and in vivo, uses. Sialyl-$Le^a$ and di-sialyl-$Le^a$ are type 1 carbohydrate chains (i.e., have a Galβ1-3GlcNAc polylactosamine unit structure) and sialyl-$Le^x$ is a type 2 carbohydrate chain (i.e., has a Galβ1-4 GlcNAc polylactosamine unit structure. A number of cancer cells, such as colorectal and pancreatic, have a prevalence of type 1 carbohydrate chains including sialyl-$Le^a$ and di-sialyl-$Le^a$. Other cancer cells, such as breast, lung and ovarian, have a prevalence of type 2 carbohydrate chains including sialyl-$Le^x$.

Regardin in vitro aspects, as noted above, there are disclosed methods for inhibiting the binding of cancer cells to endothelia in a biological preparation. Representative examples of biological preparations include blood vessel and/or lymph vessel endothelia in combination with a malignancy. The endothelia and the malignancy may be in the form of tissue or cells removed from an organism, or cultured cells. In one embodiment, the method comprises incubating a biological preparation, which contains malignant cells expressing sialyl-$Le^a$, di-sialyl-$Le^a$ or sialyl-$Le^x$ and endothelial cells expressing a selectin, with an effective amount of at least one agent, such as an antibody, saccharide or glycoconjugate as described above. In another embodiment, the method comprises incubating malignant cells with at least one enzyme inhibitor that inhibits the biosynthesis of sialyl-$Le^a$ or di-sialyl-Le by the cells. Suitable enzyme inhibitors include inhibitors of glycosyltransferases. Representative examples of inhibitors for glycosyltransferases include inhibitors for fucosyltransferases (e.g., as described by Palcic et al., J. Biol. Chem. 264:17174-17181, 1989), for N-acetylglucosaminyltransferases (e.g., as described by Palcic et al., J. Biol. Chem. 265:6759-6769, 1990), and for sialyltransferases (e.g., as described by Broquet et al., J. Neurochem. 54:388-394, 1990; Karaivanova. et al., Cancer Biochem. Biophys. 11:311-315, 1990).

In another embodiment, the method comprises incubating malignant cells expressing sialyl-$Le^a$ or di-sialyl-$Le^a$, with at least one enzyme that renders the carbohydrate on these cells unable to bind selectins. Suitable enzymes include glycosidases. Representative examples of glycosidases are sialidases (Rosen et al., Science 228:1005-1007, 1985) and fucosidases (Kobata, Methods in Enzymology 83:625-631, 1982). Enzymes possessing enhanced specificity and desirable characteristics can be detected using appropriate neoglycoproteins and anti-carbohydrate antibodies.

There is also disclosed the use of the compositions described above in methods for inhibiting metastasis in a warm-blooded animal such as a human. In one embodiment, at least one agent, such as an antibody, saccharide or glycoconjugate as described above, is used to inhibit metastasis. In another embodiment, the composition comprises at least one enzyme inhibitor (as described above) that inhibits the biosynthesis of sialyl-$Le^a$ or di-sialyl-$Le^a$ by malignant cells. In another embodiment, the composition comprises an enzyme that alters sialyl-$Le^a$ or di-sialyl-$Le^a$ of malignant cells expressing sialyl-$Le^a$ or di-sialyl-$Le^a$ such that malignant cells are incapable of binding to a selectin. It will be evident to those skilled in the art how to determine the optimal effective dose for a particular agent, enzyme itor, or enzyme (e.g., based upon in vitro and in vivo studies in non-human animals). A variety of routes of administration may be used. Typically, administration will be intravenous, intracavitory (e.g., in pleural or peritoneal cavities), or in the bed of a resected tumor.

An agent may be administered as a pharmaceutical composition, i.e., in combination with a pharmaceutically acceptable carrier or diluent, such as physiological saline. It will be recognized by those skilled in the art that an agent and a composition may be prepared in a sterile form. Moreover, an agent may be administered in combination with an immunotherapeutic or chemotherapeutic agent. When such a combination is desired, each substance may be administered sequentially, simultaneously, or combined and administered as a single composition. Diagnostic techniques, such as CAT scans for tumors, may be performed prior to and subsequent to administration to confirm effectiveness.

There are also disclosed compositions, and methods which use the same, comprising an agent capable of reacting with both sialyl-$Le^a$ and sialyl-$Le^x$. Such agents include antibodies, microbial and mammalian carbohydrate

binding proteins, such as receptors, adhesins, toxin subunits, and soluble selectins.

The following examples are offered by way of illustration and not by way of limitation.

EXAMPLES

**EXAMPLE 1**

**GLYCOCONJUGATES AND ASSAYS**

Synthetic glycoproteins (Neoglycoproteins)

Neoglycoproteins were produced by BioCarb AB (Lund, Sweden) by chemically coupling 10-20 moles of a specific oligosaccharide to 1 mole of nonglycosylated albumin, bovine (BSA) or human (HSA). The resulting synthetic glycoprotein (neoglycoprotein) contains multiple copies of the identical carbohydrate sequence, thereby producing a well characterized, mutivalent glycoconjugate which is extremely effective for studying carbohydrate-protein interactions. Depending on the size of the oligosaccharide, three different chemical spacer arms were used to couple the oligosaccharides to proteins 1) p-aminophenyl (PAP); 2) aminophenylethyl (APE); and 3) acetyl phenylene diamine were used to couple the shorter oligosaccharides to albumin since they will retain the anomeric configuration of the reducing sugars which may be involved in a potential binding site. APD was used to couple the larger sugars to protein by reductive amination, which converts the reducing sugar to an aminoalditol. These reduced sugars are designated by parenthesis in the APD conjugate presented in Table I.

**TABLE I**

| Name | Structure |
|---|---|
| LNF I | Fucα1-2Galβ1-3GlcNACβ1-3Galβ1-4(Glc) (H-type 2) |
| LNF II (Le$^a$) | Galβ1-3GlcNAcβ1-4(Glc)<br>4<br>\|<br>Fucα1 |
| LNF III (Le$^x$) | Galβ1-4GlcNAcβ1-3Galβ1-4(Glc)<br>3<br>\|<br>Fucα1 |
| sLNFII (sLe$^a$) | Neu5Acα2-3Galβ1-3GlcNAcβ1-3Galβ1-4(Glc)<br>4<br>\|<br>Fucα1 |
| sLNFIII (sLe$^x$) | Neu5Acα2-3Galβ1-4GlcNAcβ1-3Galβ1-4(Glc)<br>3<br>\|<br>Fucα1 |
| LSTa | Neu5Acα2-3Galβ1-3GlcNAcβ1-3Galβ1-4(Glc) |
| LSTc | Neu5Aca2-6Galβ1-4GlcNAcβ1-3Galβ1-4(Glc) |
| 3' Sialyllactose | Neu5Acα2-3Galβ1-4(Glc) |
| 6' Sialyllactose | Neu5Acα2-6Galβ1-4(Glc) |

Monoclonal Antibodies

The monoclonal antibodies employed include the following. HECA-452, a rat IgM [anti-CLA (Picker et al., J. Immunol. 145:3247-3255, 1990)] (Duijvestijn et al., Am. J. Path. 30:147-155, 1988); MECA-79, a rat IgM control [anti-peripheral lymph node addressin (Streeter et al., J. Cell Biol. 107:1853-1862, 1988)]; RB6-2C2, rat IgM control [Coffman and Weissman, J. Exp. Med. 153:269, 1981]; CL2 (anti-ELAM-1) (Picker et al., Nature 349:796-799, 1991), mouse $IgG_1$, kindly supplied by C. Wayne Smith (Houston, TX); Dreg-56, mouse $IgG_1$ [anti-human LECAM-1, (Kishimoto et al., Proc. Natl. Acad. Sci. USA 87:2244-2248, 1990)]; CSLEXI (TT-19, anti-sLNFIII) (Fukushima et al., Cancer Res. 44: 5279-5286, 1984), a mouse IgM, kindly given by P. Terasaki (UCLA); and 1H10 (anti-sialyl-$Le^a$), a mouse $IgG_1$ developed by BioCarb.

Direct Binding of Antibodies to Synthetic Glycoproteins (Neoglycoproteins)

Synthetic glycoproteins were coated onto microtiter plates by filling each well with 100 ng of the neoglycoprotein in 100 μl of 0.15 M sodium chloride, 0.01 M sodium phosphate, 0.1% sodium azide, pH 7.4, (PBS-azide) overnight at 4˚C. Standard enzyme-linked immunoassays (ELISA) were then performed on the solid phase carbohydrate structures using the appropriate antibody diluted to 10 μg/ml.

Production of ELAM-1 cDNA transfected cell lines

L1-2/pMRB107 cells (L1-$2^{ELAM-1}$) were prepared by transfecting the ELAM-1 gene into the murine pre-B cell line L1-2 (Gallatin et al., Nature 304:30-34, 1983). A cDNA clone encoding ELAM-1 was obtained from a cDNA library made from activated human umbilical vein endothelial cell cultures by polymerase chain reaction (PCR) amplification. The ELAM-1 gene was inserted downstream of the hCMV promoter in pMRB101 [a derivative of EE6 which contains the E. coli gpt gene (Mulligan.and Berg, Proc. Natl.. Acad. Sci. USA 78:2072, 1981; Stephens and Corbett, N.A.R. 17: 7110, 1989)]. DNA was introduced into L1-2 cells by electroporation and the cells selected for resistance to mycophenolic acid. A population of cells staining brightly for ELAM-1 were selected by FACS and cloned by limiting dilution. These cells are ELAM-$1^{hi}$ LFA-$1^{mod}$ $CD45^{hi}$ $CD44^{neg}$ LECAM-$1^{neg}$, differing from the parent cell line or control vector transfectants only in their expression of ELAM-1. L1-2/pMRB101 (L1-$2^{vector}$) cells are a similarly transformed derivative of L1-2 transfected with pMRB101 and lacking ELAM-1 expression.

Cell binding assays

One hundred microliter samples of each synthetic glycoconjugate in phosphate buffered saline (PBS), pH 7.2, were absorbed onto glass wells of 8-chamber slides (LabTek) for two hours at RT. For some experiments glass slides were pre-coated with rabbit anti-human serum albumin (Sigma) at 200μg/ml overnight at 4˚C and washed with PBS prior to the addition of the glycoconjugate. After blocking with 5% NBS/ 10mM HEPES/Dulbecco' s Modified Eagles Medium (DMEM), pH 7.0 (CM), L1-$2^{ELAM-1}$ or L1-$2^{vector}$ cells were applied to each well (1.5 x $10^6$/0.15 ml in CM). After a 25 minute incubation at RT on a rotating shaker at 50 rpm, the tops of the wells were removed and the slides washed 3x in DMEM and then fixed by incubation in 1.5% glutaraldehyde (Kodak)/DMEM. Three to six 100 x fields were counted for each data point.

Inhibition of Binding of ELAM-1 Containing Cells by Compounds

One hundred and twenty nanograms of sialyl-$Le^a$-HSA or sialyl-$Le^x$-HSA dissolved in 100 μl of phosphate-buffered saline were absorbed per well of an 8 chambered glass (LabTek) slide for 2 hours at room temperature. During this period, L1-$2^{ELAM-1}$ cells were pre-incubated for 20 minutes on ice with increasing concentrations of sialyl-$Le^a$-HSA at $10^7$ cells/ml. After washing and blocking the wells in Complete Medium (CM, 5% normal bovine serum, 10 mM HEPES, pH 7.0, DMEM), L1-$2^{ELAM-1}$ cells pre-incubated with compounds were added (1 x $10^7$ cells/ml) and incubated at room temperature while rotating at 50 rpm. After 25 minutes, slides were washed 3 times in Dulbecco's Modified Eagles Medium (DMEM) and then fixed in 1.5% glutaraldehyde/DMEM (Figure 5). The above experiments were repeated using a higher concentration of sialyl-$Le^a$-HSA to coat the wells (500 ng/100 μl) and different soluble multivalent compound (sialyl-$Le^a$-PA) for inhibition (Figure 6).

Inhibition of Intercellular Adhesion by Compounds

Normal human neutrophils or peripheral blood mononuclear cells (PBMC) (1-2 x $10^6$/ml) are incubated in CM for 30 minutes at room temperature while rotating at 50 rpm on a layer of COS cells transfected with ELAM-1 cDNA.

After washing, the binding of neutrophils is determined by directly counting the number of neutrophils bound per transfected COS cell. For PBMC, non-adherent cells are removed by washing with DMEM and then adherent cells are removed by washing with a solution of 5 mM EDTA, 5 mM EGTA in PBS. Binding of monocytes is assessed by counting the number of adherent and non-adherent cells and determining the number of monocytes by their distinctive light-scatter profile with FACS analysis and the number of $CLA^+$ lymphocytes by staining with the anti-CLA mAb HECA-452 (Picker et al., Nature 349:796-799, 1991). Neutrophils and/or PBMC are pre-incubated with sialyl-$Le^a$-HSA or other compounds prior to incubation on the layer of ELAM-1 cDNA transfected COS cells. Inhibition of intercellular adhesion is determined as a percentage calculated by:

$$\frac{\text{number of bound cells in control - number of bound cells in test} \times 100}{\text{number of bound cells in control}}$$

Binding of Lymphocytes or LECAM-1 cDNA Transfectants to High Endothelial Venules

The interaction of the peripheral lymph node homing receptors (LECAM-1) with high endothelial venules is measured in a frozen section assay in which a suspension of lymphocytes and/or LECAM-1 transfected cell lines are incubated on frozen sections of lymphoid tissues for 20-30 minutes at 7°C (Stamper and Woodruff, J. Exp. Med. 144: 828, 1976; Butcher et al., Eur. J. Immunol. 10:556, 1980) while rotating at 50 rpm. After glutaraldehyde fixation, the number of cells bound per HEV is determined microscopically. Sialyl-$Le^a$-HSA and other compounds are pre-incubated with lymphocytes or transfected cell lines, including LECAM-1 and ELAM-1 transfected L1-2 cells, prior to the assay, and the ability of the compounds to inhibit intercellular adhesion is determined as described above.

EXAMPLE 2

CARBOHYDRATE STRUCTURES RECOGNIZED BY ELAM-1

The sensitive binding assay described in Example 1 uses cells permanently transfected with ELAM-1 cDNA. The mouse pre-B cell line, L1-2, transfected with ELAM-1 cDNA (L1-$2^{ELAM-1}$), but not vector control cDNA, L1-$2^{vector}$ expresses very high levels of ELAM-1. The ELAM-1 expressed by these cells is functional as L1-$2^{ELAM-1}$ cells are adhesive for neutrophils and this adhesion is blocked by anti-ELAM-1 monoclonal antibodies. When added to glass slides coated with various synthetic glycoconjugates, L1-$2^{ELAM-1}$ cells bound selectively to sialyl-$Le^a$ and sialyl-$Le^x$ neoglycoproteins, but not to a number of other glycoconjugates (see Table I for structures). L1-$2^{ELAM-1}$ cells also bound, albeit more weakly, to $Le^a$ neoglycoprotein. The binding to $Le^a$ is significant as L1-$2^{ELAM-1}$ cells bound poorly to $Le^x$ and not at all to the glycoconjugates prepared with the structural analogs such as LNF I. That L1-$2^{ELAM-1}$ cells did not bind other monosialylated carbohydrates, such as 3'SL, 6'SL, LSTa or LSTc demonstrates that the binding to sialyl-$Le^a$ and sialyl-$Le^x$ is not due to non-specific charge effects, but rather reflects specific structural features of these oligosaccharides. The low level of binding of ELAM-1 transfectants to $Le^a$ is consistent with an essential role of fucose in recognition, but shows that neuraminic acid (also known as sialic acid) also plays a key role.

Hard Sphere Exo-Anomeric (HSEA) Calculations

Conformational models of the oligosaccharides in solution were obtained by HSEA calculations. Hydroxyl groups are represented by the oxygen atoms. A fixed bond angle of 117° was used for the glycosidic linkages. The energy calculated by a HSEA potential (Bock, Pure Appl. Chem. 55:605-622, 1983), was minimized using simultaneous variation of dihedral angles (multi-dimensional binary chop). This algorithm shows a slow convergence near a local minimum when compared to other methods utilizing the first and second derivative, but has the advantage of allowing a large initial search area of the conformational space, whereby the chances of finding the lowest local minima increases. Other applications of this program are described in Kumlien et al. (Arch. Biochem. Biophys. 269:678-689, 1989) and Wreslander et al. (Glycoconjugate J. 7:85-100, 1990).

**Carbohydrate Structures that Inhibit the Binding of ELAM-1 Dependent Intercellular Adhesion**

Sialyl-$Le^a$-HSA in solution blocks 10% of binding of ELAM-1 transfected cells (L1-$2^{ELAM-1}$) to either immobilized sialyl-$Le^x$-HSA or immobilized sialyl-$Le^a$-HSA. As binding to either carbohydrate structure is blocked by sialyl-$Le^a$-HSA,

only one carbohydrate-binding site exists in ELAM-1 which recognizes a carbohydrate domain common to both sialyl-$Le^a$ and sialyl-$Le^x$.

## Graphic Representation of the Carbohydrate Epitope for ELAM-1

The dihedral angles for sialyl-$Le^a$ and sialyl-$Le^x$ hexassacharide determined by the HSEA calculations are presented in Table II. It should be noted that these are theoretical approximations of the native conformation and the disclosure is not restricted to these bond angles. The dihedral angles are specified by the designation of the four atoms defining it. These 4-character designations are made up of the chemical symbol, 2 characters for the number in the monosaccharide (and possible extra specification, e.g., to distinguish atoms of the same type bonded to the same carbon), and the number of the monosaccharide residue in the oligosaccharide. The last number is defined below:

```
1           2            3           4            5
      Neu5Acα2-3Galβ1-*GlcNAcβ1-3Galb1-4Glc
                              #
                              |
                            Fucα1

                             6
*=3 in case of sialyl-Le^a; 4 in case of sialyl-Le^x.
#=4 in case of sialyl-Le^a; 3 in case of sialyl-Le^x.
```

**TABLE II**

| Dihedral Angle | | | Sialyl-$Le^a$(˚) | Sialyl-$Le^x$ (˚) |
|---|---|---|---|---|
| C1 1 - C2 | 1 - 02 | 1 - C3 2 | 188.6240387 | 189.0001221 |
| C2 1 - 02 | 1 - C3 | 2 - H3 2 | 350.8763428 | 349.9999695 |
| H1 2 - C1 | 2 - 01 | 2 - C 3 | 51.3739777 | 53.2507896 |
| C1 2 - 01 | 2 - C | 3 - H* 3 | 15.3727636 | 8.8746433 |
| H1 3 - C1 | 3 - 01 | 3 - C3 4 | 57.8722878 | 57.8755035 |
| Cl 3 - 01 | 3 - C3 | 4 - H3 4 | 350.6306458 | 350.6243591 |
| H1 4 - Cl | 4 - 01 | 4 - C4 5 | 55.3822517 | 55.4999657 |
| Cl 4 - 01 | 4 - C4 | 5 - H4 5 | 2.6262217 | 1.6283444 |
| H1 6 - C1 | 6 - 01 | 6 - C# 3 | 49.7558937 | 49.8749161 |
| C1 6 - 01 | 6 - C# | 3 - H# 3 | 18.8635368 | 23.9994240 |
| 09 1 - C9 | 1 - C8 | 1 - C7 1 | 178.1011200 | 178.2247772 |
| 08 1 - C8 | 1 - C7 | 1 - C6 1 | 299.6979065 | 300.0729675 |
| 07 1 - C7 | 1 - C6 | 1 - H6 1 | 180.4196625 | 182.0449371 |
| 06 2 - C6 | 2 - C5 | 2 - H5 2 | 303.0703125 | 300.8233032 |
| 06 3 - C6 | 3 - C5 | 3 - H5 3 | 289.2540588 | 294.3686523 |
| 06 4 - C6 | 4 - C5 | 4 - H5 4 | 306.4527283 | 306.4491882 |
| 06 5 - C6 | 5 - C5 | 5 - H5 5 | 296.1771851 | 178.0553131 |
| H6A6 - C6 | 6 - C5 | 6 - H5 6 | 179.6257324 | 179.8791046 |

* = 3 in case of sialyl-$Le^a$; 4 in case of sialyl-$Le^x$

\# = 4 in case of sialyl-$Le^a$; 3 in case of sialyl-$Le^x$.

EMIN (sialyl-$Le^a$) = -15.7628746 kcal/mol

EMIN (sialyl-$Le^x$) = -14.9528790 kcal/mol

For control purposes the full program output is presented, while the relevant accuracy for comparison with

experimental data cannot be expected to be higher than ± for the angles. The error in the HSEA energy value can be expected to lie in the first decimal, when given in kcal/mol. These energy values are of interest when comparing different potential functions, etc., but does not lend itself easily to comparison with energies determined from experiments. A large negative value does, however, show that the attractive van der Waals forces dominate the calculations, giving support for the use of the HSEA approximation (positive energies indicate strong steric forces that may distort bond lengths and angles, which are assumed constant in HSEA). The resulting structures are represented graphically in Figure 1.

The calculations show high similarity in the corresponding dihedral angles for the two structures, also at the bonds with different linkage between the N-acetyl-glucosamine and the fucose and sialic acid residues, respectively. The different dihedral angles for the hydroxymethyl group in the 6-position of the glucose residue at the reducing terminal (296.2° and 178.1°) is a result of the very nearly equal energies for this molecular group after a rotation of 120°. As this group is far away from the linkages differing between slialyl-$Le^a$ and sialyl-$Le^x$, its direction is of no importance for the conformational structure in this region. Computer-generated images of the structures are represented graphically in Figure 1. The conformations indicate that the structures show a high degree of similarity in both the non-reducing and reducing terminal parts, respectively. In particular, the structures of the terminal carbohydrate sequence up to but not including the N-acetyl group on the internal GlcNAc residue, show a high degree of homology and may represent the domain recognized by both ELAM-1 and the monoclonal antibody HECA-452. This area of structural homology is particularly useful for the design of potential anti-inflammatory drugs.

Examples of other carbohydrate-binding proteins that recognize type 1 and type 2 chain isomers are the antibodies $E_1$23-48 and $E_1$66-18 which bind the blood group B antigen (Hansson et al., J. Biol. Chem. 258:4091, 97, 1983) and the lectin, Griffonia simplicifolia IV, which recognizes both $Le^b$ and $Le^y$ antigens (Spohr et al., Can. J. Chem. 63:2644-52, 1985).

The recognition of the sialyl-$Le^a$ antigen and sialyl-$Le^x$ antigen, by ELAM-1, may be of pathologic importance. Mucins containing these structures are elevated in the sera of cancer patients, including gastrointestinal, pancreatic, and breast cancer patients (Magnani et al., J. Biol. Chem. 257:14365-369, 1982; Magnani et al., Cancer Res. 43: 5481-92, 1983). Preliminary experiments indicate that some sialyl-$Le^a$- and sialyl-$Le^x$-containing mucins are recognized by ELAM-1 transfectants. By interacting with ELAM-1 on venules in acute and chronically inflamed tissues and interfering with the recruitment of leukocytes to these locations, these mucins secreted by tumors may contribute to the immunodepressed state of cancer patients.

## EXAMPLE 3

## CARBOHYDRATE STRUCTURES RECOGNIZED BY LECAM-1

### Production of LECAM-1 cDNA transfected cells

A human LECAM-1 cDNA transfected cell line (L1-$2^{LECAM-1}$) was prepared by transfecting the LECAM-1 gene into the murine pre-B cell line Ll-2 (Gallatin et al., Nature 304:30-34, 1983). A cDNA clone encoding LECAM-1 was obtained from a cDNA library made from peripheral blood lymphocytes by polymerase chain reaction amplification. The LECAM-1 gene was inserted downstream of the hCMV promoter in pMRB101 [a derivative of EE6 which contains the E. coli gpt gene (Mulligan and Berg, Proc. Natl. Aca. Sci. USA 78:2072-2076, 1981; Stephens and Cockett, N.A.R. 7: 7110, 1989)]. DNA was introduced into L1-2 cells by electroporation and the cells selected for resistance to mycophenolic acid. A population of cells staining brightly for LECAM-1 were selected by FACS and cloned by limiting dilution. These cells are LECAM-$1^{hi}$ LFA-$1^{mod}$ $CD45^{hi}$ $CD44^{neg}$, differing from the parent cell line or control vector transfectants only in their expression of LECAM-1. L1-2/PMRB101 (L1-$2^{vector}$) cells are a similarly transformed derivative of L1-2 transfected with pMRB101 and lack LECAM-1 expression.

### Cell binding assay

One hundred microliter samples of each neoglycoconjugate in phosphate buffered saline (PBS), pH 7.2, were absorbed onto glass wells of 8-chamber slides (LabTek) for two hours at room temperature. After blocking with 5% NBS, 10 mM HEPES, Dulbecco's Modified Eagles Medium (DMEM), pH 7.0 (CM), L1-$2^{LECAM-1}$, L1-$2^{vector}$ or L1-$2^{ELAM-1}$ cells were applied to each well (1.5 x $10^6$ cells in 0.15 ml CM). Mouse lymphocytes isolated from mesenteric lymph nodes were also tested at 3 x $10^6$ cells in 0.15 ml. In some cases, cells were pre-incubated with monoclonal antibody MEL-14 (Gallatin et al., 1983, supra) at 150 $\mu$g/ml/$10^7$ cells and washed prior to testing. After a 25-minute incubation at room temperature on a rotating shaker at 50 rpm, the tops of the wells were removed and the slides washed 3x in DMEM and then fixed by incubation in 1.5% glutaraldehyde (Kodak) in DMEM. Three to six 100 x fields were counted for each data point and the average and standard error are reported. Data reported are from representative experiments which were

performed 2-5 times with similar results.

Lymphocytes bind to Sialyl-$Le^x$ and Sialyl-$Le^a$ containing neoglycoconjugates via LECAM-1

The determination that LECAM-1 cross-reacts with these ELAM-1 ligands (described in Example 1) was performed by repeating this adherence assay with L1-2 cells transfected with human LECAM-1 cDNA (L1-$2^{LECAM-1}$) as well as with normal mouse lymphocytes which express high levels of mouse LECAM-1. Mouse lymphocytes bound sialyl-$Le^x$ and sialyl-$Le^a$, but not LNF I, $Le^a$, $Le^x$ or neoglycoconjugates containing 3' sialyllactose, 6' sialyllactose, or LSTa. Binding of mouse lymphocytes was blocked by anti-mouse LECAM-1 MAB MEL-14 (Gallatin et al., 1983, supra) demonstrating that the adhesion observed is via LECAM-1 (Figure 8). L1-$2^{LECAM-1}$ cells all bind slightly better to sialyl-$Le^a$ than sialyl-$Le^x$ containing conjugates over a wide range of cell concentrations; both ELAM-1 and LECAM-1 appear to display similar relative binding abilities to these two carbohydrate ligands (Figure 7).

It is evident from the above results, that compositions can be employed which can be used to modulate the homing of leukocytes, particularly lymphocytes, to sites of inflammation. These compounds can be readily prepared by conventional ways and can be effective for the treatment of a variety of diseases, both prophylactically and therapeutically.

From the foregoing, it will be evident that, although specific embodiments of the invention have been described herein for purposes of illustration, various modifications as may be made without deviating from the scope of the invention as defined in the claims.

## Claims

1. A composition comprising a pharmaceutically acceptable carrier or diluent in combination with a compound comprising Neu5Acα2-3Galβ1-x[Fucα1-y]R, wherein x is 3 and y is 4, and R is 1,2-cyclohexanediol; 1,2-cyclohexanolamine; 1,2-cyclopentanediol; 2,3-dihydroxypyran; or 3,4-dihydroxypyran; or with a compound with the structure Neu5Acα2-3Galβ1-x[Fucα1-y]R, wherein x is 3 and y is 4, and R is glucose; or with the compound Sialyl-$Le^a$ with the structure:

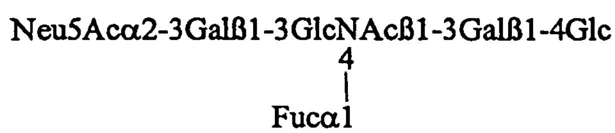

2. A composition according to claim 1 wherein the compound comprises Neu5Acα2-3Galβ1-x[Fucα1-y]R, wherein x is 3 and y is 4, and R is 1,2-cyclohexanediol; 1,2-cyclohexanolamine; 1,2-cyclopentanediol; 2,3-dihydroxypyran; or 3,4-dihydroxypyran.

3. A composition according to claim 1 wherein the compound is Neu5Acα2-3Galβ1-x[Fucα1-y]R, wherein x is 3 and y is 4, and R is glucose.

4. A compound comprising Neu5Acα2-3Galβ1-x[Fucα1-y]R, wherein x is 3 and y is 4, and R is 1,2-cyclohexanediol; 1,2-cyclohexanolamine; 1,2-cyclopentanediol; 2,3-dihydroxypyran; or 3,4-dihydroxypyran.

5. A compound or composition according to any one of claims 1-4 wherein the compound or the compound of the composition is bonded to a physiologically acceptable polymer.

6. A compound or composition according to claim 5 wherein the polymer is a serum albumin.

7. A compound or composition according to any one of claims 1-6, for use in a method of modulating the binding of leukocytes or platelets to endothelial cells.

8. A compound or composition according to any one of claims 1-6, for use in a method for inhibiting the infiltration of leukocytes into an inflammation site of a host.

9. A compound or composition according to claim 8 wherein the leukocytes are lymphocytes.

**10.** A compound or composition according to any one of claims 1-6, for use in a method for inhibiting the spread of malignant cells expressing sialyl-Le$^a$, di-sialyl-Le$^a$ or sialyl-Le$^x$, to secondary sites.

**11.** A compound according to claim 10 wherein the spread of malignant cells to secondary sites is by hematogenous metastases.

**12.** The use of a compound or composition according to any one of claims 1-6, for the manufacture of a medicament to treat inflammation, leukocyte infiltration or cancer.

**13.** The compound or composition of any one of claims 1 to 6 for use as a medicament.

**Patentansprüche**

**1.** Zusammensetzung umfassend einen pharmazeutisch akzeptablen Träger oder ein solches Verdünnungsmittel zusammen mit einer Verbindung, die Neu5Acα2-3Galβ1-x[Fucα1-y]R umfasst, wobei x gleich 3 und y gleich 4, und R 1,2-Cyclohexandiol; 1,2-Cyclohexanolamin; 1,2-Cyclopentandiol; 2,3-Dihydroxypyran; oder 3,4-Dihydroxypyran ist; oder mit einer Verbindung mit der Struktur Neu5Acα2-3Galβ1-x[Fucα1-y]R, wobei x gleich 3 und y gleich 4, und R Glucose ist; oder mit der Verbindung Sialyl-Le$^a$ mit der Struktur:

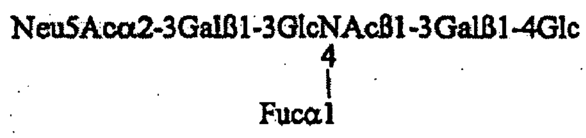

**2.** Zusammensetzung nach Anspruch 1, **dadurch gekennzeichnet, dass** die Verbindung Neu5Aca2-3Galβ1-x[Fuca1-y]R umfasst, wobei x gleich 3 und y gleich 4 und R 1,2-Cyclohexandiol; 1,2-Cyclohexanolamin; 1,2-Cyclopentandiol; 2,3-Dihydroxypyran; oder 3,4-Dihydroxypyran ist.

**3.** Zusammensetzung nach Anspruch 1, **dadurch gekennzeichnet, dass** die Verbindung Neu5Aca2-3Galβ1-x [Fucα1-y]R ist, wobei x gleich 3 und y gleich 4 und R Glucose ist.

**4.** Verbindung, die Neu5Acα2-3Galβ1-x[Fucα1-y]R umfasst, wobei x gleich 3 und y gleich 4 und R 1,2-Cyclohexandiol; 1,2-Cyclohexanolamin; 1,2-Cyclopentandiol; 2,3-Dihydroxypyran; oder 3,4-Dihydroxypyran ist.

**5.** Verbindung oder Zusammensetzung nach einem der Ansprüche 1 - 4, **dadurch gekennzeichnet, dass** die Verbindung oder die Verbindung der Zusammensetzung mit einem physiologisch akzeptablen Polymer verbunden ist.

**6.** Verbindung oder Zusammensetzung nach Anspruch 5, **dadurch gekennzeichnet, dass** das Polymer ein Serumalbumin ist.

**7.** Verbindung oder Zusammensetzung nach einem der Ansprüche 1 - 6 zur Verwendung in einem Verfahren zur Modulation der Bindung von Leukozyten oder Blutplättchen an Endothelzellen.

**8.** Verbindung oder Zusammensetzung nach einem der Ansprüche 1 - 6 zur Verwendung in einem Verfahren zur Inhibition der Infiltration von Leukozyten in eine Entzündungsstelle eines Wirtsorganismus.

**9.** Verbindung oder Zusammensetzung nach Anspruch 8, **dadurch gekennzeichnet, dass** die Leukozyten Lymphozyten sind.

**10.** Verbindung oder Zusammensetzung nach einer der Ansprüche 1 - 6 zur Verwendung in einem Verfahren zur Inhibition der Ausbreitung von malignen Zellen, die Sialyl-Le$^a$, Disialyl-Le$^a$ oder Sialyl-Le$^x$ darstellen, auf sekundäre Stellen.

**11.** Verbindung nach Anspruch 10, **dadurch gekennzeichnet, dass** die Ausbreitung von malignen Zellen auf sekundäre Stellen durch hämatogene Metastasen erfolgt.

**12.** Verwendung einer Verbindung oder Zusammensetzung nach einem der Ansprüche 1 - 6 zur Herstellung eines Medikaments zur Behandlung von Entzündungen, leukozytärer Infiltration oder Krebs.

**13.** Verbindung oder Zusammensetzung nach einem der Ansprüche 1 bis 6 zur Verwendung als Medikament.

**Revendications**

**1.** Composition comprenant un support ou diluant pharmaceutiquement acceptable en combinaison avec un composé comprenant Neu5Acα2-3Galβ1-x[Fucα1-y]R, où x est 3 et y est 4, et R est le 1,2-cyclohexanediol; la 1,2-cyclohexanolamine; le 1,2-cyclopentanediol; le 2,3-dihydroxypyrane; ou le 3,4-dihydroxypyrane; ou avec un composé ayant la structure Neu5Acα2-3Galβ1-x[Fucα1-y]R, où x est 3 et y est 4, et R est le glucose; ou avec le composé Slalyl-$Le^a$ ayant la structure :

Neu5Acα2-3Galβ1-3GalcNAcβ1-3Galβ1-4Glc
4
|
Fucα1

**2.** Composition selon la revendication 1 où le composé comprend Neu5Acα2.3Galβ1-X[Fucα1-y]R, où x est 3 et y est 4, et R est le 1,2-cyclohexanediol.; la 1,2-cyclohexanolamine; le 1,2-cyclopentanediol ; le 2,3-dihydrexypyrane ; ou le 3,4-dihydroxypyrane.

**3.** Composition selon la revendication où le composé est Neu5Aco2-3Galβ 1-x[Fucα 1-y]R, où x est 3 et y est 4, et R est le glucose.

**4.** Composé comprenant Neu5Acα2-3Galβ1-x[Fucα1-y]R, où x est 3 et y est 4, et R est le 1,2-cyclohexanediol ; la 1,2-cyclohexanolamine le 1,2-cyclopentanediol; le 2,3-dihydroxypyrane ; ou le 3,4-dihydroxypyrane.

**5.** Composé ou composition selon l'une quelconque des revendications 1-4 où le composé ou le composé de la composition est lié à un polymère physiologiquement acceptable.

**6.** Composé ou composition selon la revendication 5 où le polymère est une sérumalbumine.

**7.** Composé ou composition selon l'une quelconque des revendications 1-6 destiné à être utilisé dans un procédé de modulation de la liaison de leucocytes ou de plaquettes à des cellules endothéliales.

**8.** Composé ou composition selon l'une quelconque des revendications 1-6 destinè à être utilisé dans un procédé pour inhiber l'infiltration de leucocytes dans un site d'inflammation d'un hôte.

**9.** Composé ou composition selon la revendication 8 où les leucocytes sont des lymphocytes.

**10.** Composé ou composition selon l'une quelconque des revendications 1-6 destiné à être utilisé dans un procédé pour inhiber la propagation de cellules malignes exprimant sialyl-$Le^a$, di-sialyl-$Le^a$ ou sialyl-$Le^a$ à des sites secondaires.

**11.** Composé selon la revendication 10 où la propagation de cellules malignes à des sites secondaires est par des métastases hématogènes,

**12.** Utilisation d'un composé ou composition selon l'une quelconque des revendications 1-6 pour la fabrication d'un médicament pour traiter l'inflammation, l'infiltration des leucocytes ou le cancer.

**13.** Composé ou composition selon l'une quelconque des revendications 1 à 6 destiné â être utilisé comme médicament.

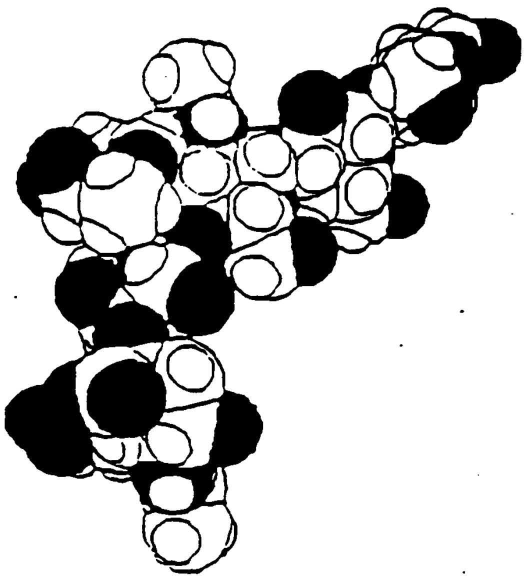

Sialyl $Le^x$ Hexasaccharide

FIG. 1A

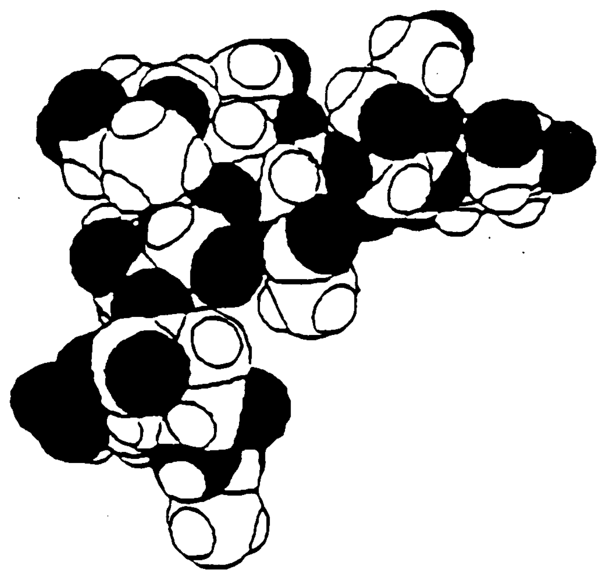

Sialyl $Le^a$ Hexasaccharide

FIG. 1B

Figure 2

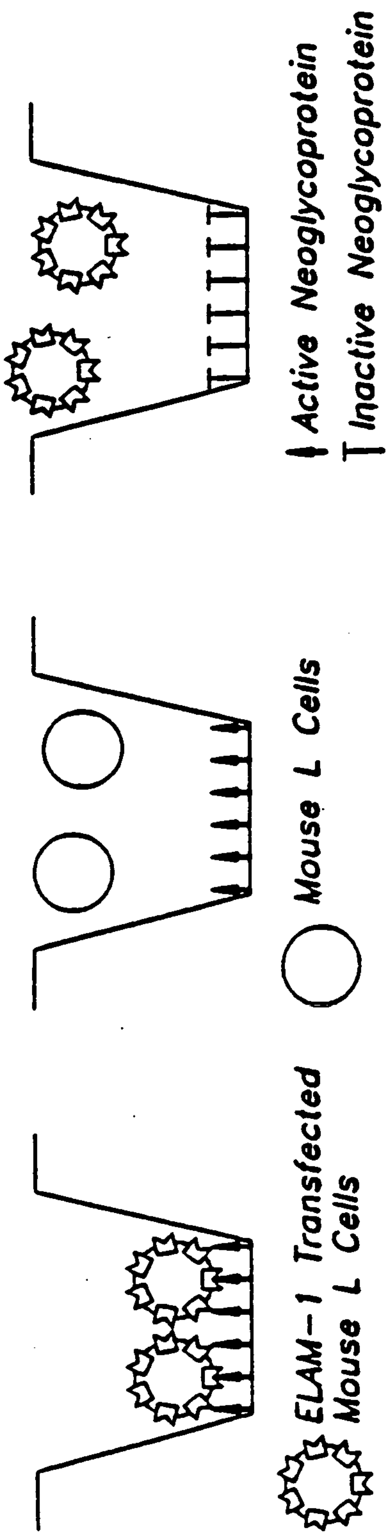
ELAM-1 Transfected
Mouse L Cells
Mouse L Cells
Active Neoglycoprotein
Inactive Neoglycoprotein

*Figure 3*

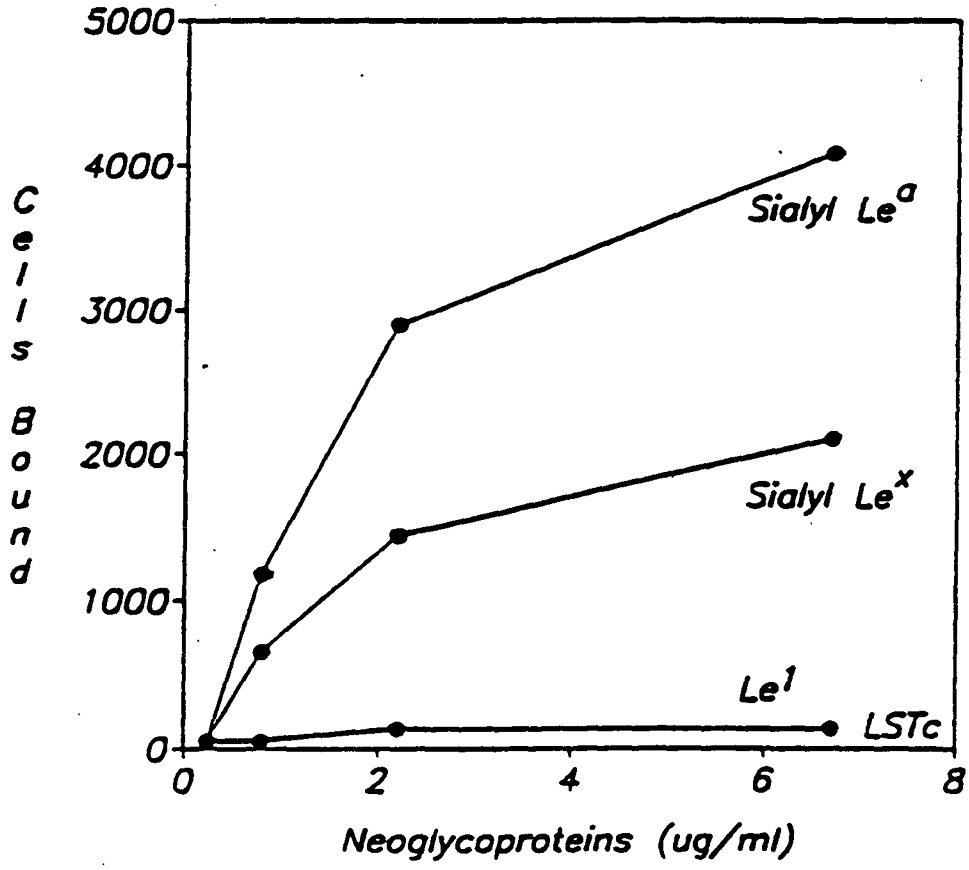

5000
4000
3000
2000
1000
0
Cells Bound
Sialyl Le^a
Sialyl Le^x
Le^1
LSTc
0
2
4
6
8
Neoglycoproteins (ug/ml)

*Figure 4*

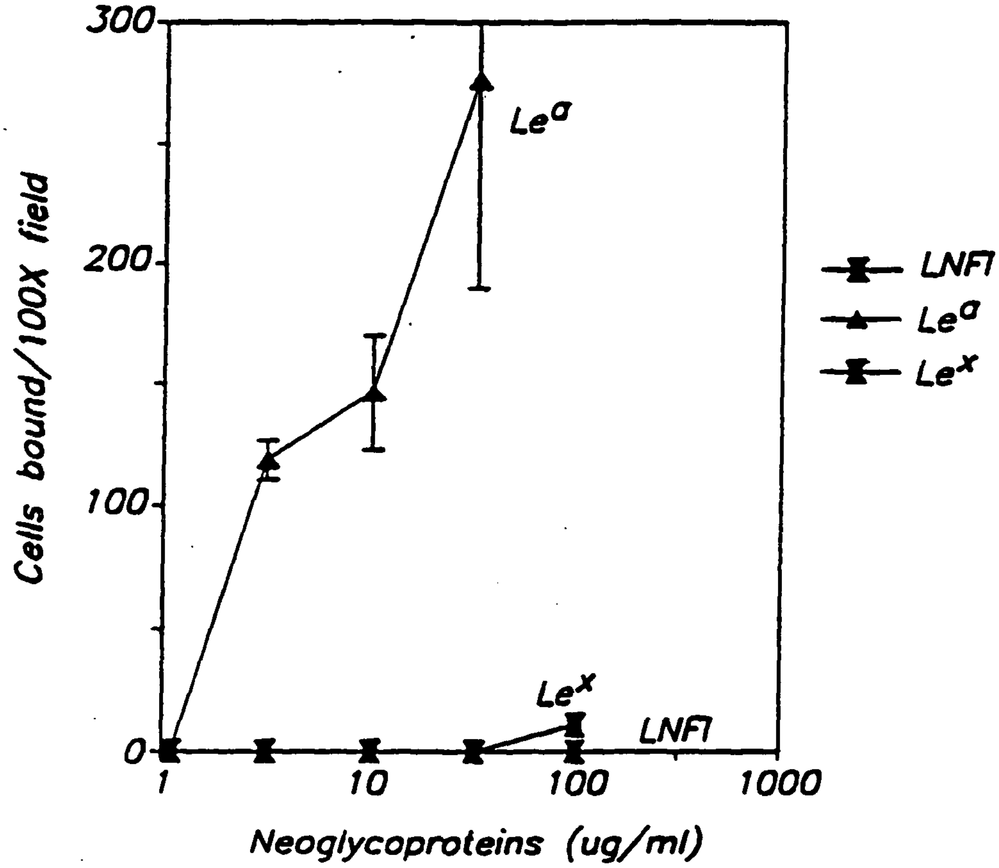
300
200
100
0
1
10
100
1000
Cells bound/100X field
Neoglycoproteins (ug/ml)
$Le^a$
$Le^x$
LNFI
LNFI
$Le^a$
$Le^x$

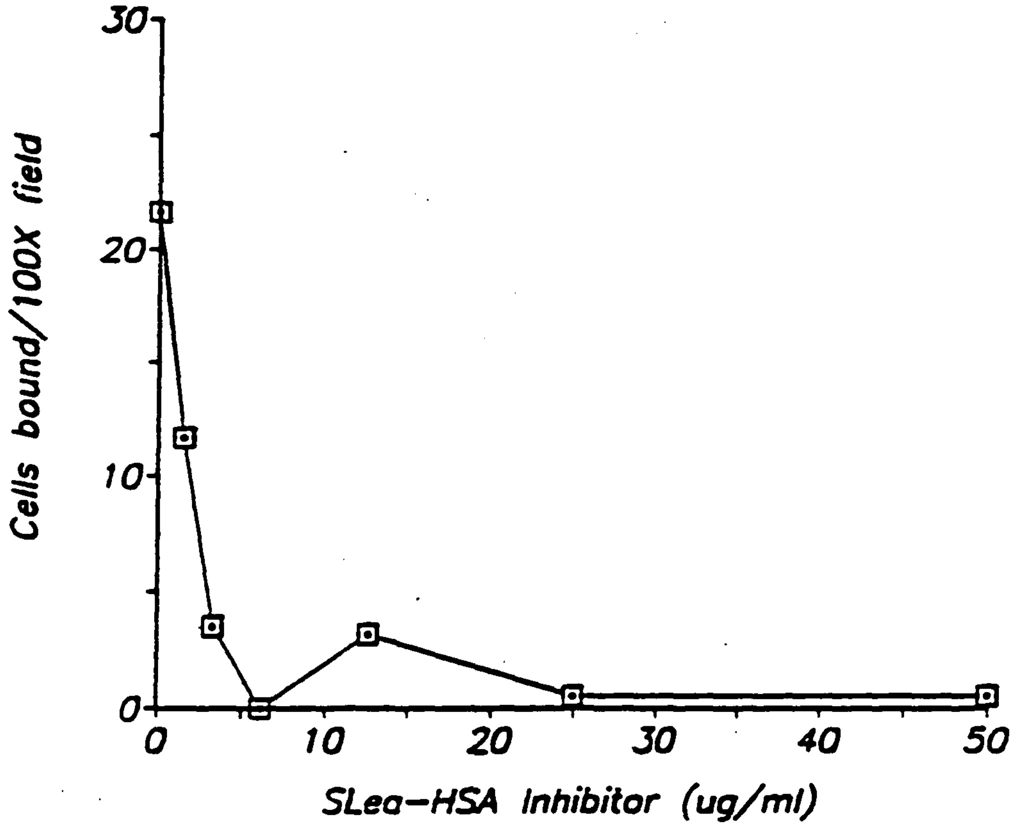
30
20
10
0
Cells bound/100X field
0
10
20
30
40
50
SLea–HSA Inhibitor (ug/ml)

*Figure 5*

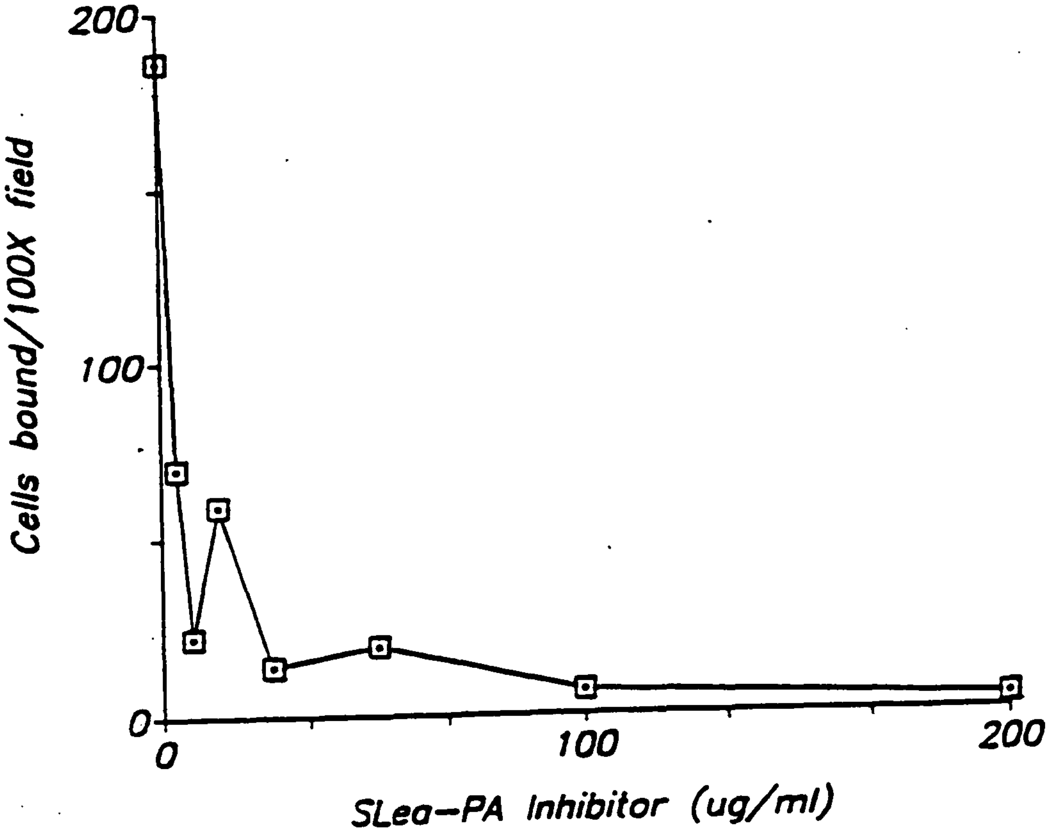
200
100
0
Cells bound/100X field
0
100
200
SLea-PA Inhibitor (ug/ml)

Figure 6

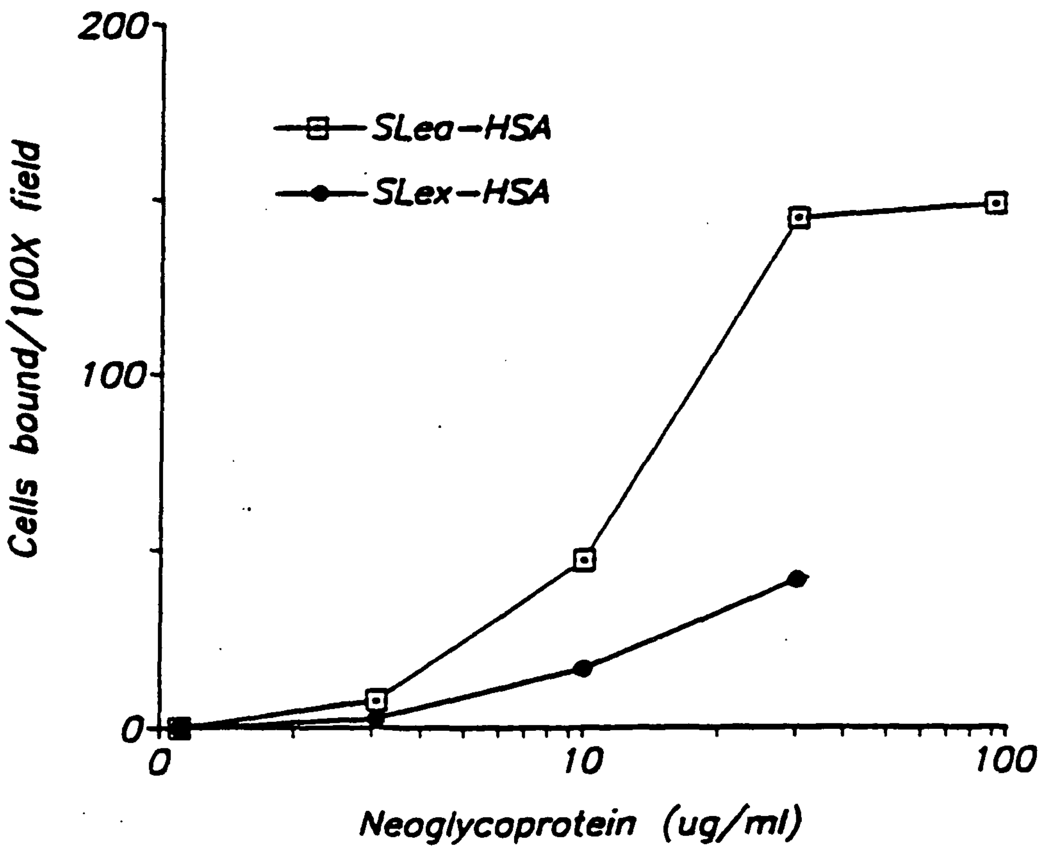
200
100
0
Cells bound/100X field
SLea–HSA
SLex–HSA
0
10
100
Neoglycoprotein (ug/ml)

Figure 7

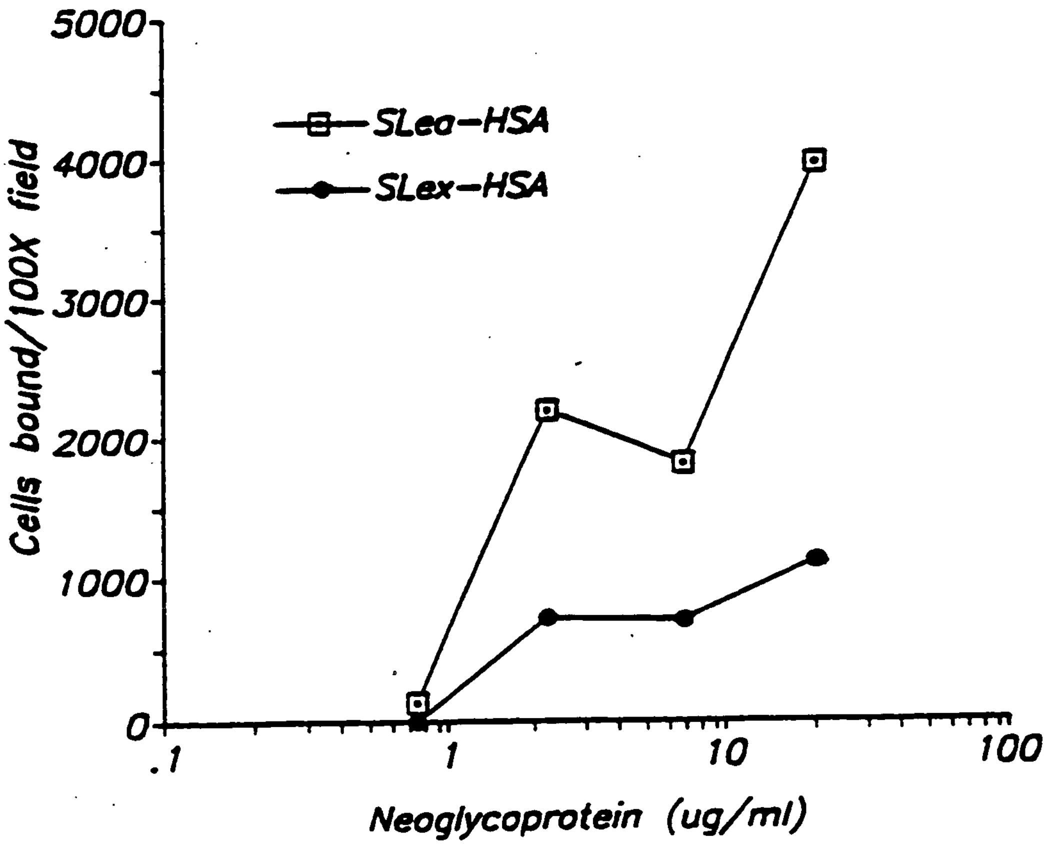
5000
4000
3000
2000
1000
0
SLea-HSA
SLex-HSA
Cells bound/100X field
.1
1
10
100
Neoglycoprotein (ug/ml)

*Figure 8*